# EUROPEAN PATENT SPECIFICATION

(11) **EP 3 381 517 B1**
(45) Date of publication and mention of the grant of the patent: **16.03.2022**
(21) Application number: 18163201.9
(22) Date of filing: 21.03.2018
(51) Int. Cl.: A61Q 19/00, A61K 9/127, A61K 8/14, A61K 31/685

(54) **PHOSPHOLIPID THREE-DIMENSIONAL VESICULAR AGGREGATES SCATTERED IN ALCOHOLIC MIXTURES WITH NO OR LOW WATER CONTENT, THEIR PREPARATION AND USE IN FORMULATIONS FOR TOPICAL APPLICATION**
VESIKULÄRE, DREIDIMENSIONALEN AGGREGATEN VON PHOSPHOLIPIDEN, AUSGESTREUT IN ALCOHOLISCHEN MISCHUNGEN MIT KEINEM ODER GERINGEM WASSERINHALT, IHRE HERSTELLUNG UND VERWENDUNG FÜR ZUSAMMENSETZUNGEN FÜR TOPISCHE ANWENDUNG.
AGRÉGATS VESICULAIRES TRIDIMENSIONELS DE PHOSPHOLIPIDES, DIFFUSÉS DANS DES MILEUX ALCOOLIQUES AVEC PAS D'EAU OU UN CONTENU D'EAU TRÈS BAS, LEUR PRÉPARATION ET LEUR USAGE DANS DES FORMULATIONS POUR USAGE TOPIQUE.

(30) Priority: 29.03.2017 IT 201700034725
(43) Date of publication of application: 03.10.2018
(73) Proprietor: UNIVERSITA' DEGLI STUDI DI CAGLIARI, 09124 Cagliari (IT)
(72) Inventor: MANCA, Maria Letizia, 09124 Cagliari (IT); MANCONI, Maria, 09124 Cagliari (IT); FADDA, Anna Maria, 09124 Cagliari (IT)
(74) Representative: Primiceri, Maria Vittoria

(56) References cited:
- EP-A1- 3 025 732
- WO-A1-96/31196
- WO-A2-2010/022873
- US-A1- 2005 158 389
- US-A1- 2008 145 415
- CASTANGIA INES ET AL: "Effects of ethanol and diclofenac on the organization of hydrogenated phosphatidylcholine bilayer vesicles and their ability as skin carriers", JOURNAL OF MATERIALS SCIENCE: MATERIALS IN MEDICINE, SPRINGER NEW YORK LLC, UNITED STATES, vol. 26, no. 3, 26 February 2015 (2015-02-26), pages 1-9, XP035457431, ISSN: 0957-4530, DOI: 10.1007/S10856-015-5443-1 [retrieved on 2015-02-26]
- CASTANGIA INES ET AL: "Faceted phospholipid vesicles tailored for the delivery ofSantolina insularisessential oil to the skin", COLLOIDS AND SURFACES. B, BIOINTERFACES, ELSEVIER, AMSTERDAM, NL, vol. 132, 22 May 2015 (2015-05-22), pages 185-193, XP029189590, ISSN: 0927-7765, DOI: 10.1016/J.COLSURFB.2015.05.025
- MANCA MARIA LETIZIA ET AL: "Molecular arrangements and interconnected bilayer formation induced by alcohol or polyalcohol in phospholipid vesicles", COLLOIDS AND SURFACES. B, BIOINTERFACES, vol. 117, 12 March 2014 (2014-03-12), pages 360-367, XP028643378, ISSN: 0927-7765, DOI: 10.1016/J.COLSURFB.2014.03.010

## Description

### Technical Field

The present invention relates to three-dimensional vesicle aggregates substantially formed by phospholipids dispersed in solutions comprising or consisting of a mixture of alcohols and/or polyalcohols and/or glycols and/or polyglycols and/or polyoxyethylenes and possibly a small percentage of water, to the preparation thereof, and to the use thereof in pharmaceutical and/or cosmetic formulations for topical application. In particular, the present invention relates to three-dimensional aggregates of nanometric phospholipid-based vesicles (nanovesicles), obtained by starting from various/different phospholipid molecules and at least one pharmacologically and/or cosmetically active principle, preferably water-insoluble or poorly water-soluble, but also water-soluble (if so desired), which are dispersed in a carrier substantially formed by mixtures having a different composition of alcohols, polyalcohols and/or polyoxyethylenes, in the presence of a small quantity or in the total absence of water (from 0 to 25% by volume [v/v]; preferably 0 to 20% [v/v]. The invention further relates to the preparation of said aggregated phospholipid-based vesicles and to the use thereof in topical compositions for carrying said pharmaceutical and/or cosmetic active substances, preferably those that are water-insoluble or poorly water-soluble.

### Background Art

Phospholipids are well-known amphiphilic substances formed by a hydrophilic "*head*" and two hydrophobic "*tails*"*.* In water, these molecules aggregate spontaneously as bilayers, in which the heads face towards the external aqueous environment and the tails arrange themselves within the bilayer against one another, so as to minimize contact with water. For thermodynamic reasons, the bilayers fold to form lamellar vesicles, known as liposomes, which may be formed either by a single bilayer (uni-lamellar) or by different concentric bilayers (multi-lamellar). Phospholipids are commonly dispersed into water to obtain liposomes, or into substantially aqueous mixtures, e.g. hydroalcoholic or hydroglyceric mixtures with a water content ≥40%, in order to obtain ethosomes, glycerosomes, or the so-called PEVs (*Penetration Enhancer Vesicles*), i.e. vesicles containing substances capable of facilitating the passage of active principles through biological membranes. All these phospholipid-based vesicles are individually and homogeneously dispersed in the aqueous carrier and are generally stable over time due to their high surface charge (negative or positive), which creates electrostatic repulsion of the vesicles, thus preventing aggregation and fusion thereof. Such systems are commonly used in the pharmaceutical and/or cosmetic industry in order to carry active substances of natural or synthetic origin, and can enhance bioavailability and/or release to the site of action.

Liposomes can carry both hydrophilic drugs and lipophilic drugs. Hydrophilic drugs are solubilized in the aqueous compartments, in the central core or in the aqueous spaces between the lamellae, until the saturation concentration of the drug in water is achieved. Lipophilic drugs are intercalated in the phospholipid bilayer, but, if their concentration saturates the bilayer, part of the drug will precipitate. In order to increase the "loading" capacity of liposomes, different strategies have been used, which include increasing the lipid concentration, using small percentages of surfactants together with phospholipids, and, above all, using co-solvents miscible with water (hereinafter referred to, for simplicity, as *"aqueous co-solvents*")*.* These *aqueous co-solvents,* consisting of, for example, ethyl alcohol in ethosomes, glycerol in glycerosomes, and polyalcohols in PEVs, are mixed with water in percentages that may vary from 1% to 60% (v/v), as is well known to the person of average skill in the art, and can increase the solubility of several lipophilic drugs in the aqueous carrier, and hence the "loading" capacity of the vesicles. Furthermore, due to the presence of alcohols or polyalcohols behaving as cutaneous absorption promoters, these vesicles are generally capable of increasing the dermal and/or transdermal absorption of said lipophilic drugs, which are generally poorly absorbed by topical administration.

As an alternative to lamellar structures, phospholipids may form flat single layers (or "*organogels*") when a phase immiscible in water, e.g. oil or ether, is added to the aqueous dispersion. Or, by adding high concentrations of surfactants and/or oily substances, it is possible to induce the formation of so-called inverse micelles. These systems can increase the dermal and/or transdermal absorption of drugs. However, these systems can only be obtained by using precise quantities of the various components. What is more, when these systems are present *in vivo* in biological liquids, the resulting dilution and change in their composition may cause the system itself to fail. In addition, these systems imply the use of non-ionic surfactants and apolar solvents, which are not always as biocompatible and/or ecological/eco-sustainable as phospholipids, alcohols and polyalcohols. At any rate, the different states of aggregation of phospholipids have been widely used as carriers of different drugs.

The most-important criticalities of liposome-like vesicles are their limited capacity of loading some active molecules, especially lipophilic ones, and their poor stability in dispersion, so that the vesicles will tend to aggregate and possibly fuse together over time, thereby releasing the active substances being carried and changing their structure and dimensions. Another property of many phospholipid-based vesicles is their interaction and fusion with biological membranes. This phenomenon may pose a limitation if the membrane is the skin and the aim is percutaneous absorption, in that it may promote accumulation of active substances in the first layers of the membranes themselves, without allowing them to actually pass therethrough. A further limitation of phospholipid-based vesicles, when they have to be applied onto the skin, is the low viscosity of their dispersions, which, since they are as fluid as water, are unable to remain on the site of application for a long time. In order to overcome this problem, vesicle dispersions can be incorporated into a preformed gel, which however often limits the actual transfer of the vesicles from the gel to the skin, thus reducing the effectiveness of the system compared to liquid vesicle dispersions.

To the present inventors' knowledge, no publications and/or patents are known which describe the preparation of phospholipid-based vesicles by using mixtures of alcohols and polyalcohols, and possibly a small quantity of water, at any rate not exceeding 25% (v/v).

### Technical Problem

Therefore, those skilled in the art strongly feel the need for new, stable phospholipid-based vesicle systems which can effectively carry significant concentrations of active substances by topical administration, such systems being preferably water-insoluble or poorly water-soluble and not suffering from the drawbacks that are typical of the analogous liposome-like systems previously described. In particular, said innovative systems should be able to "*load*" high concentrations of active agents, should have very good stability in dispersion, and should facilitate the passage of said active agents through all biological membranes, the skin in the first place.

Reference is also made to Castangia et al., "J. of Materials Science", 26(3), 2015, pages 1-9 and to Castangia et al., "Colloids and Surfaces", vol. 132, 2015, pages 185-193.

The present invention aims at providing an adequate solution to the above-described technical problem.

### Summary of the Invention

The present inventors have now found out that appropriate dispersions of phospholipids in a mixture of alcohols, polyalcohols and/or polyoxyethylenes, whether or not in the presence of a small percentage of water (0 to 25% v/v), form three-dimensional vesicular aggregates that can advantageously be used for carrying active substances of natural or synthetic origin in pharmaceutical and/or cosmetic compositions, and can provide an adequate solution to the technical problem highlighted above.

Therefore, it is one object of the present invention to provide dispersions of three-dimensionally aggregated phospholipid-based nanovesicles containing at least one pharmacologically active and/or cosmetic principle, wherein said phospholipid-based vesicles are dispersed in a mixture substantially consisting of alcohols, polyalcohols and/or polyoxyethylenes, as set out in the appended independent claim.

It is another object of the present invention to provide a method for preparing said dispersions of aggregated vesicles as described above, as set out in the appended independent claim.

The present invention further relates to the use of said aggregated vesicles as described above, as set out in the appended independent claim.

Preferred embodiments of the present invention are set out in the appended dependent claims.

The preferred embodiments of the present invention will be described in the following description merely by way of example and without limitation of the broad applicability of the present invention, which will be immediately apparent to the person skilled in the art.

### Brief Description of the Drawings

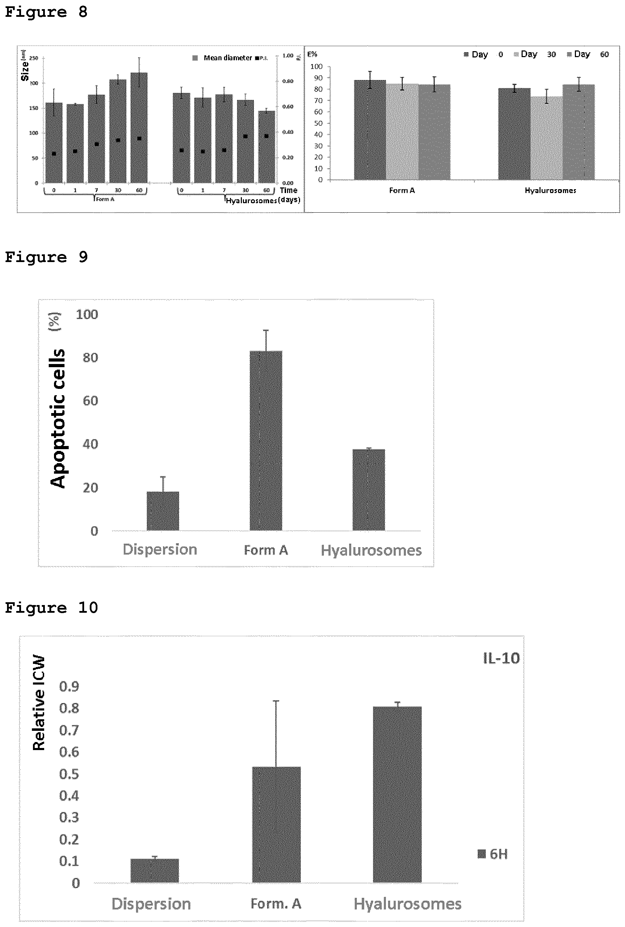

The present invention will now be described by way of non-limiting example by illustrating some preferred embodiments thereof. For this purpose, in order to better highlight the potentialities and peculiarities of the aggregated phospholipid-based vesicles of the present invention, reference will also be made to the annexed drawings, which present some aspects and features of said nanovesicles, which have been used, merely by way of example, for carrying clotrimazole, which is a lipophilic synthetic drug with anti-mycotic activity, curcumin, which is a strongly lipophilic natural substance with anti-oxidizing and anti-inflammatory activity, and baicalin, which is a poorly water-soluble natural substance with photoprotective activity.
**Figure** 1 shows some representative images, taken with a transmission electron microscope, of Formulations A and B of aggregated vesicles incorporating clotrimazole.
**Figure** 2 shows some representative images, taken with a transmission electron microscope at low temperature, of Formulations A and B of aggregated vesicles incorporating clotrimazole, previously diluted with water (1/500 v/v).
**Figure** 3 shows the values, measured during 90 days of preservation at 25±1°C, of the average size, polydispersity index (PI) and zeta potential of the different Formulations (A, B, C and D) of aggregated vesicles incorporating clotrimazole.
**Figure** 4 shows the quantity of clotrimazole (%) accumulated in the corneous layer (SC), in the epidermis (Ep), in the dermis (D), and the total quantity of drug accumulated in the skin after 8 hours of treatment with the different formulations (A, B, C and D) of aggregated nanovesicles. A dispersion of clotrimazole in ethanol, glycerol and water and a commercial cream containing the same concentration of active principle (Canesten^{®}) were used as comparisons. The mean values ± standard deviation obtained from six repetitions are indicated.
**Figure** 5 shows the viability values of keratinocytes (%) incubated with the different Formulations (A, B, C and D) of aggregated nanovesicles incorporating clotrimazole.
As a comparison, the dispersion of clotrimazole in ethanol, glycerol and water was used.
The mean values ± standard deviation calculated from at least six measurements are indicated.
**Figure** 6 shows the values of the anti-mycotic activity of clotrimazole on two different strains of Candida (*Albicans* and *Kruis*). The different Formulations (A, B, C and D) of aggregated nanovesicles or, as a comparison, the dispersion of clotrimazole in ethanol, glycerol and water and a commercial cream containing the same concentration of active principle (Canesten^{®}) were used. The mean values ± standard deviation are indicated.
**Figure** 7 shows a representative image, taken with a transmission electron microscope, of aggregated nanovesicles incorporating curcumin.
**Figure** 8 shows the variation, occurred during 60 days, of the size, polydispersity index (PI) and incorporation efficiency (E) of the aggregated vesicles of the present invention (Form A) or hyalurosomes incorporating curcumin.
**Figure** 9 shows the synoviocyte pro-apoptosis values of patients suffering from rheumatoid arthritis, treated with curcumin scattered in ethanol, glycerol and water or incorporated in aggregated vesicles (Form A) or in hyalurosomes. The mean values ± standard deviation obtained from six repetitions are indicated.
   The graph of **Figure 10** shows the IL-10 values obtained by treating the synoviocytes of patients suffering from rheumatoid arthritis with curcumin dispersed in ethanol, glycerol and water or incorporated in aggregated vesicles (Form A) or in hyalurosomes. The mean values ± standard deviation obtained from six repetitions are indicated.

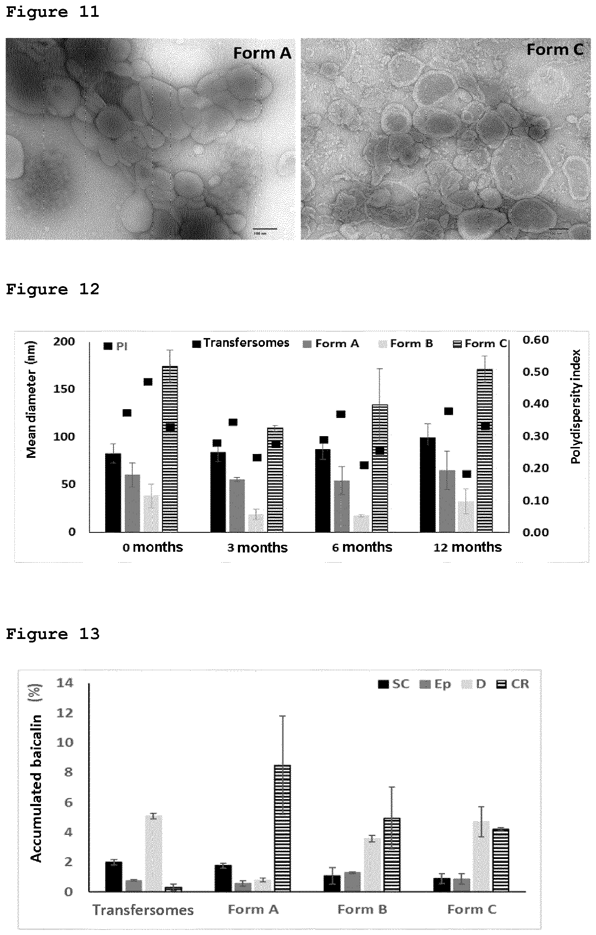

**Figure 11** shows some representative images, taken with a transmission electron microscope, of Formulation A (left image) and Formulation C (right image) of aggregated phospholipid-based vesicles carrying baicalin.
**Figure 12** shows the mean diameter and polydispersity index (PI) values of transfersomes or Formulations (A, B, C) of aggregated vesicles carrying baicalin. The samples had been preserved at 5°C for 12 months. The mean values ± standard deviation obtained from six repetitions are indicated.
**Figure 13** shows the percentages of accumulated baicalin (%) in the corneous layer (SC), in the epidermis (Ep), in the dermis (D) and in the receptor compartment (CR) after 8 hours of application of the same, carried in transfersomes or in Formulations (A, B, C) of aggregated vesicles. The mean values ± standard deviation obtained from six repetitions are indicated.

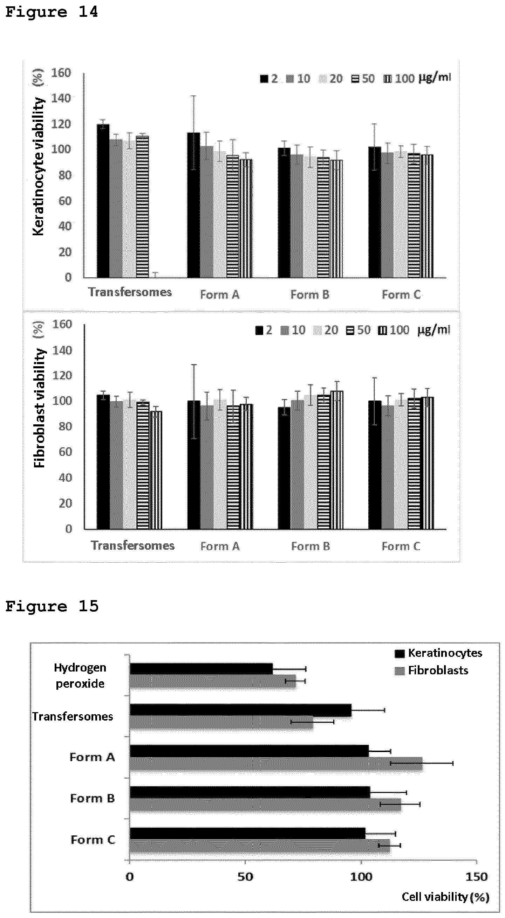

**Figure 14** shows the viability values of keratinocytes (upper graph) and fibroblasts (lower graph) treated for 48 hours with baicalin carried in transfersomes or in Formulations (A, B, C) of aggregated vesicles. The mean values ± standard deviation obtained from six repetitions are indicated.
**Figure 15** shows the viability values of keratinocytes and fibroblasts treated for 4 hours with hydrogen peroxide or with hydrogen peroxide and baicalin (5 µg/ml) carried in transfersomes or in Formulations (A, B, C) of aggregated vesicles. The mean values ± standard deviation obtained from six repetitions are indicated.

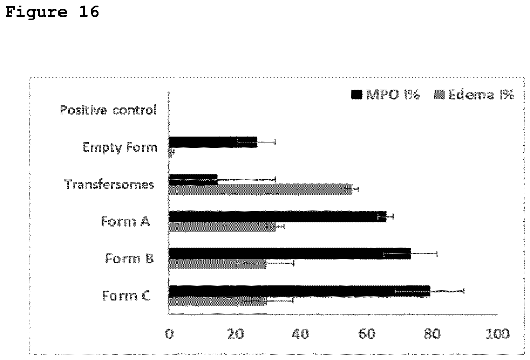

**Figure 16** shows the percentages of inhibition of edema and myeloperoxidase (MPO) measured in mouse skin, inflamed (positive control) with TPA (12-0-tetradecanoylphorbol-13-acetate) and then treated locally with baicalin carried in transfersomes or in Formulations (A, B, C) of aggregated vesicles. The mean values ± standard deviation obtained from at least four repetitions are indicated.

### Detailed Description of the Invention

The present invention relates to dispersions of three-dimensionally aggregated nanometric vesicles, formed by phospholipids dispersed in a mixture substantially consisting of alcohols and polyalcohols and/or polyoxyethylenes and intended for pharmaceutical and/or cosmetic topical application, comprising or consisting of:
a) at least one phospholipid preferably selected from those described below and/or derivatives and/or mixtures thereof;
b) at least one pharmaceutical and/or cosmetic active principle of natural or synthetic origin, and/or a natural substance and/or a natural extract of vegetable or animal origin, and/or a mixture thereof;
c) a carrier/dispersing agent consisting of a mixture of aqueous co-solvents such as alcohols and/or polyalcohols and/or polyoxyethylenes and a quantity of water of 0 to 25% (v/v), relative to the total volume of the dispersing carrier.

In one embodiment of the invention, water amounts to 0% v/v, relative to the total volume of the dispersing carrier.

In one embodiment of the invention, water amounts to 1 to less than 25% v/v, relative to the total volume of the dispersing carrier.

In the present invention, the at least one phospholipid is usually selected among one or more natural or synthetic phospholipids, whether pure or blended; for example, from the group including soy or egg lecithin, phosphatidylcholine at different degrees of purity, whether hydrogenated or non-hydrogenated, phosphatidylethanolamine, phosphatidylserine, phosphatidylglycerol, phosphatidylinositol, dimyristoylphosphatidylcholine, dipalmitoylphosphatidylcholine, distearoylphosphatidylcholine, palmitoylstearoylphosphatidylcholine, sphyngomyelin, hydrogenated and non-hydrogenated phospholipids derived from soy and/or mixtures thereof. Preferably, said at least one phospholipid is selected from the group including phosphatidylcholine, preferably phosphatidylcholine of hydrogenated and non-hydrogenated soy, and derivatives thereof, e.g. those mentioned above and/or mixtures thereof.

The fundamental characteristics of the phospholipids chosen for preparing the aggregated vesicles of the present invention are high biologic compatibility, stability and absence of toxicity.

The aggregated phospholipid-based vesicles of the present invention have proved to be particularly versatile and can be used as carriers of active principles, whether lipophilic or not, preferably lipophilic, and/or natural substances and/or extracts, whether of vegetable or animal origin, preferably of vegetable origin, which can be solubilized at molecular level in so-called aqueous co-solvents within the nanovesicles and/or between the lipid tails of the phospholipids that constitute them.

In the present invention, the at least one pharmaceutical and/or cosmetic active principle and/or the natural substance to be carried in the nanovesicles according to the present invention is selected, for example, from the group including antiviral drugs, vitamins, e.g. vitamin C and vitamin E, etc., active plant extracts, anti-inflammatory drugs, antihistaminic drugs, corticosteroids, antibiotics, fungicides, antiasthmatic drugs, anti-psoriasis drugs, anaesthetics, alkaloids, hydroxy acids, antioxidants, anti-acne drugs, active agents for hair regrowth, anti-tricotic agents and moisturizers, and/or mixtures thereof. Preferably, the pharmaceutical and/or cosmetic active principles of natural or synthetic origin are selected from the group including those known in the art for topical use. Preferably, the aggregated phospholipid-based nanovesicles of the present invention can be used as carriers for highly lipophilic (water-insoluble or poorly water-soluble) substances, e.g. including, without being limited to, curcumin, quercetin, resveratrol, kaempferol, diclofenac, acyclovir, trans-retinoic acid, betamethasone dipropionate, minoxidil, gentamicin, rifampicin, azoles (clotrimazole, fluconazole, etc.), tocopherol and its ester derivatives or the lipophilic derivatives of vitamin E, but also natural extracts containing hydrophilic and lipophilic substances or even poorly water-soluble substances, such as baicalin, or water-soluble substances, such as allantoin, and so on.

In the present invention, the water-miscible dispersing carrier consists of the above-mentioned mixture of co-solvents (defined as "*aqueous*" in the description of the present invention), wherein said aqueous co-solvents have been selected, by way of non-limiting example, from the group including ethanol, isopropyl alcohol, glycerol, ethylene glycol, propylene glycol, all polyethylene glycols (PEG) with different molecular weight. Preferably, said aqueous co-solvents are ethanol, glycerol and/or propylene glycol.

In one embodiment of the invention, in said mixture of aqueous co-solvents water may be totally absent (0%). Or, in another embodiment of the invention, water may be present in a small quantity ranging from 0 to 25% v/v, relative to the total quantity of the dispersing carrier.

The nanovesicular aggregate of the present invention may further comprise modifying agents and/or surfactants and/or preserving agents and/or a suitable mixture thereof.

In particular, the modifying agents are added to the formulation as stabilizers, and may be selected, for example, from the group including cholesterol, stearylamine, oleic acid, dicetylphosphate and phosphatidylglycerol, cholesterol, and/or mixtures thereof.

The surfactants may be added to the formulation in order to promote phospholipid aggregation in nanovesicles and to increase the solubility of lipophilic drugs, and may be selected, for example, from the group including polyoxyethylenes, polysorbates, polyglucoside ethers and/or mixtures thereof.

The preserving agents are selected from all those known in the art and commonly used in the pharmaceutical and/or cosmetic field for topical, preferably cutaneous, application.

In general, without however limiting the broad range and scope of application of the present invention, the dispersions of aggregated vesicles of the present invention have the average composition indicated, for convenience, in the following two Tables, respectively designated as Table a) and Table b). Table a) shows the possible average composition of the nanovesicles, and Table b) shows the possible average composition of the dispersing carrier.

**Table a) - Composition of the nanovesicles of the invention**

| **Phospholipids** | **Active principles** | **Natural extracts or substances** | **Modifiers and/or surfactants** | **Preservatives** | **Dispersing** carrier |
|---|---|---|---|---|---|
| (% w/v) | (% w/v) | (% w/v) | (% w/v) | (% w/v) | (% w/v) |
| **1 - 45** | **0 - 10** | **0 - 10** | **0 - 10** | **0 - 1** | **60 - 99** |

**Table b) - Composition of the aqueous dispersing carrier**

| **Alcohols** (% v/v) | **Glycols** (% v/v) | **Polyalcohols or polyoxyethylenes** (% v/v) | **Water** (% v/v) |
|---|---|---|---|
| **0 - 70** | **0 - 70** | **0 - 70** | **0 - 25** |

Preferably, the phospholipid component (expressed as % w/v, relative to the total volume of the mixture of solvents) ranges from 1% to 45%; more preferably, from 2% to 40%; even more preferably, from 5 to 25%.

Preferably, the quantity of active principle(s) (expressed as % w/v) ranges from 0% to 10%; more preferably, from 0.1% to 5%; even more preferably, from 0.5% to 3%. Preferably, the quantity of modifiers and/or surfactants (expressed as % w/v) ranges from 0% to 10%.

Preferably, the quantity of preservatives (expressed as % w/v) ranges from 0% to 1%. Preferably, the quantity of dispersing carrier (expressed as % w/v) ranges from 60% to 99%.

The dispersing carrier is preferably formed by a quantity of alcohols and/or glycols and/or polyalcohols and/or polyoxyethylenes (expressed as % v/v) ranging, for each one of them, from 0% to 70%; more preferably from 10% to 60%; even more preferably from 20% to 50%.

Preferably, the quantity of water in the dispersing carrier (expressed as % v/v) ranges from 0% to ≤25%; more preferably from 1% to 20%; even more preferably from 2% to 15%.

The aggregated phospholipid-based nanovesicles of the present invention may have a size ranging, for example, from 60 to 800 nm; preferably from 70 to 500 nm; more preferably from 80 to 400 nm. When diluted in water, they maintain the vesicular structure but lose the three-dimensional superstructure, dispersing as single (non-aggregated) vesicles having a size ranging from 30 to 250 nm. Moreover, the aggregated phospholipid-based vesicles of the present invention can be easily incorporated into formulations intended for topical application, such as, for example, creams, pastes, gels, oleogels, lotions, emulsions, suspensions, and the like. Said aggregated phospholipid-based nanovesicles may also be loaded on suitable transdermal plasters to ensure controlled release of the incorporated active agent.

It is therefore a further object of the present invention to provide a pharmaceutical and/or cosmetic composition for topical use comprising the aggregated phospholipid-based nanovesicles, preferably in association with one or more pharmaceutically or cosmetically acceptable excipients or adjuvants known to be particularly useful for the cutaneous release of the active principles.

The present invention also concerns, therefore, the use of the three-dimensionally aggregated phospholipid-based nanovesicles and/or of the above-described pharmaceutical and/or cosmetic compositions thereof for therapeutic and cosmetic treatment of skin disorders. Said disorders include, by way of non-limiting example, acne and cicatritial alterations, ulcers and/or keloid, atopical and seborrheic dermatitis, and some autoimmune diseases like psoriasis. Non-limiting examples of cosmetic treatments based on the use of the aggregated phospholipid-based nanovesicles of the present invention include treatments with antiageing function, with antioxidant function, with draining activity, with hydrating and nourishing function and/or with soothing function, e.g. post-peeling treatments.

A further aspect of the present invention relates to a method for preparing said dispersions of aggregated phospholipid-based nanovesicles of the present invention. Said method comprises or consists of the following steps:
a) preparing, under stirring, the dispersing carrier formed by a homogeneous mixture containing the different aqueous co-solvents, any hydrophilic surfactants, and possibly water, if the latter is present;
b) mixing, under stirring, the homogeneous mixture obtained in step a) with at least one phospholipid and at least one active principle and/or a natural substance and/or an extract, until hydration of said phospholipid and formation of a colloidal system structured as aggregated phospholipid-based nanovesicles are achieved;
c) sonicating said colloidal system obtained in step b) by using 10 to 200 cycles (each cycle consisting of 2-10 seconds ON and 2-10 seconds OFF) with a wave amplitude of 13-16 µm; or, preferably, by using 15 to 50 cycles (each cycle consisting of 2-5 seconds ON and 2-5 seconds OFF) with a wave amplitude of 14-15 µm; or, more preferably, 20 to 30 cycles (each cycle consisting of 5 seconds ON and 2 seconds OFF) with a wave amplitude of 14-15 µm; wherein all the steps of the above-described method are carried out at a temperature below 30°C; preferably ranging from 20°C to 28°C; more preferably, at about 25°C, i.e. substantially at room temperature.

In one embodiment, in accordance with the method of the present invention, the dispersing carrier of the aggregated vesicles of the present invention may contain ethyl and/or isopropyl alcohol, preferably ethyl alcohol, in a variable concentration ranging from 10% to 70% (v/v), glycerol and/or ethylene glycol and/or polyoxyethylene glycol (or mixtures thereof) in a variable concentration ranging from 10% to 70% (v/v), water in a variable concentration ranging from 0% to 25% (v/v).

The at least one phospholipid should be used for preparing the aggregated nanovesicles of the invention in a quantity ranging from 1% to 45% (w/v); preferably from 5% to 40% (w/v): more preferably from 5% to 25% (w/v).

The method of the present invention, in comparison with those known in the art for preparing other phospholipid-based systems of vesicular nature, does not require the use of any toxic organic solvent, as is normally the case in the preparation of prior-art conventional liposomes, in order to solubilize the phospholipids and then dry them as a film. In the present invention, the drug and/or the cosmetic agent and/or the natural substance and/or the extract are added to the phospholipids, which are then hydrated by direct addition of the dispersing carrier formed by a mixture of alcohols and/or polyalcohols and/or polyoxyethylenes, and optionally water. In this manner, the nanovesicles are formed by spontaneous aggregation of the phospholipids, the velocity of which is accelerated by simply supplying energy by sonication. The composition of the mixture of alcohols, polyalcohols and water (if any), the type of phospholipid and the concentration thereof shall be determined experimentally on a time-to-time basis by means of preliminary formulation tests.

Non-ionic surfactants, such as, for example, polyethylenglycols (PEG) with different molecular weight, Tween, OramixCG 110^{®}, may possibly be added, in a variable concentration ranging from 0% to 10% (w/v), to the dispersion of the pharmaceutical and/or cosmetic active agent and the phospholipids.

### Experimental Section

The following experimental section will describe some of the characteristic aspects of the present invention, without however limiting the broad application potential thereof.

### Materials and Methods

### 1.1 Preparation of the aggregated phospholipid-based nanovesicles and comparison liposomes

The three-dimensionally aggregated phospholipid-based nanovesicles were prepared by weighing together the at least one active principle and the at least one phospholipid in the same bottle and by adding thereto the dispersing carrier previously homogeneously mixed (ethanol/glycerol/water; ethanol/glycerol/propylene glycol/water; in different percentages). The samples thus obtained were then sonicated by means of an ultrasonic disintegrator for 10-200 cycles (2-5 seconds per cycle, with pauses of 2-5 seconds), until a homogeneous and transparent dispersion was obtained.

The comparison liposomes were prepared (whenever possible) with the same method, i.e. by dispersing the components in water and then sonicating until a homogenous dispersion was obtained.

### 1.2 Characterization of the aggregated phospholipid-based nanovesicles and comparison liposomes

The actual formation of lamellar nanovesicles, whether uni-, oligo- or multi-lamellar, was confirmed through observation of the samples under a transmission electron microscope (TEM). The structure and morphology of the nanovesicles after dilution (1:500 v/v) with water was determined by using a cryogenic transmission electron microscope (cryo-TEM).

By using the Photon Correlation Spectroscopy (PCS) technique, the size and dimensional distribution of the nanovesicles were then measured. The loading efficiency of the nanovesicles was measured by calculating the concentration of the active agent in the dispersions before and after purification of the vesicles from non-incorporated active agent. Purification was conducted by dialysis.

In order to prepare the samples for observation with TEM and cryo-TEM, a drop of dispersion was caused to be absorbed on a carbon grid, and any excess was removed with filter paper. For TEM observation, the sample was colored with phosphotungstic acid (1%) and then observed and photographed with a transmission electron microscope with accelerated voltage of 80 kV; a *JEM-1010* apparatus (Jeol Europa, France) was used.

For cryo-TEM observation, the grid was vitrified by immersion in ethane maintained at the melting point. The sample was observed with a *Tecnai F20 TEM* apparatus (FEI Company) and photographed at 200 kV, at -173°C, by using a *CCD Eagle* photo camera (FEI Company).

The average size and the amplitude of dimensional distribution (PI) were measured with the PCS technique by using a *Zetasizer nano-ZS* instrument (Malvern Instrument, UK); the zeta potential was measured by using the same instrument and a Phase Analysis Light Scattering analyzer (M3-PALS), which measures the electrophoretic mobility of particles in a thermostat-controlled cell.

The loading efficiency (incorporation for lipophilic drugs and encapsulation for hydrophilic ones) was measured as a percentage of the quantity of active agent found after purification, compared to the initial quantity. The free active agent was removed by dialysis, by inserting 1-2 ml of sample into a dialysis tube, closed at both ends, and immersing it into a water bath kept at 25°C under constant stirring. The water of the bath was changed 1-4 times during the experiment, so as to be sure that all non-encapsulated/incorporated molecules could be removed; the total quantity of water used was sufficient to solubilize all the active principle or natural active agent loaded in the dialysis tube (generally 10-20 mg). The nanovesicular dispersions before and after the dialysis were diluted with methanol (1/1000), so as to break the vesicles and release all the active agent, the concentration of which was quantified by HPLC analysis by using the conditions known in the art for the substance under examination.

### 1.3 Quantitative determination of clotrimazole, curcumin and baicalin

The quantitative determination of the three molecules under examination (clotrimazole, curcumin and baicalin) was conducted with a high-resolution chromatograph (HPLC), *Alliance 2690* (Waters, Milan, Italy), equipped with a UV spectrophotometric detector, by using a wavelength λ = 211 nm for clotrimazole, λ = 427 nm for curcumin and λ = 277 nm for baicalin and a *SunFire C18* column (3.5 µm, 4.6 × 150 mm). As a mobile phase for measuring the concentration of clotrimazole, a mixture of water/methanol/acetonitrile (30:5:65 v/v/v) eluted at a velocity of 1 ml/min was used; for curcumin, a mixture of acetonitrile/water/acetic acid (94.8:5:0.2 v/v/v) was used, eluted at a velocity of 1 ml/min; for baicalin, a mixture of water and methanol (35:65 v/v) was used, eluted at a velocity of 0.5 ml/min.

### 1.4 In vitro study of cutaneous penetration/permeation

*In vitro* studies were conducted by using newborn pig skin, which was removed immediately after the animal's death, washed with abundant water and frozen at -80°C. At the time of the experiment, the skin was cut into small disks, pre-balanced for 3 hours with saline at 32°C, and then mounted in Franz cells, interposing it between the two compartments of the cells. Cells were used which had an effective diffusion area of 0.785 cm². The receptor cell was filled with saline (~6 ml of NaCl 0.9%), which was kept constantly under stirring throughout the experiment, at a thermostat-controlled temperature of 37°C in order to reach the physiological temperature of ~32°C on the skin. In the donor compartment, 100 µl of each sample were deposited, taking care of covering the whole surface of the skin. Six cells were used for each formulation. At predetermined times (2, 4, 6 and 8 hours), the receptor compartment was emptied and filled with fresh saline at a previously thermostat-controlled temperature. The collected solutions were then lyophilized, recovered with 2 ml of methanol and subsequently analyzed with HPLC to measure the concentration of the molecules under examination. At the end of the 8 hours the skin was washed and dried with filter paper. The corneous layer was then separated by applying a piece of adhesive tape (Master-Aid, RollTex) on the skin for 10 seconds, applying a light pressure and then pulling it off together with the corneous layer. The tape was cut into small pieces, which were collected in a test tube containing 2 ml of methanol. The dermis was separated from the epidermis by using a scalpel, and both layers were cut into small pieces and transferred into a test tube containing 2 ml of methanol. In order to ensure the proper extraction of the molecules under examination from the different layers of the skin, the methanolic solutions were sonicated (2 cycles of one minute each) with a *Soniprep 150* (MSE Crowley, UK). The samples were analyzed with HPLC to determine the quantity of active principle that was present in the different layers of the skin.

### 1.5. Effect of curcumin and formulations thereof on inflammation caused by rheumatoid arthritis

### Culture of synoviocytes

Synovial tissue was obtained during a synovectomy operation. The tissue was incubated in DMEM culture medium (*Dulbecco Modified Eagle Medium*) and digested through Collagenase A for 2 hours at 37°C in order to isolate the synoviocytes. Once isolated, the synoviocytes were maintained in culture in a humidified incubator at 37°C in the presence of 5% CO₂, by using DMEM containing 10% fetal bovine serum (FBS), 100 U/ml of penicillin, 100 mg/ml of streptomycin and L-glutamine. The culture medium was changed every 2-3 days, and the cells were generally divided every 10-15 days, until confluence was achieved.

### Apoptosis Test

The synovial cells were maintained in culture in DMEM medium, enriched with synovial liquid, in order to reproduce the *in vivo* conditions during the pathologic process, and then treated for 24 hours with curcumin in dispersion or incorporated into hyalurosomes or into aggregated nanovesicles (Form A). Apoptotic cells were detected by using an apoptosis detection kit (*Annessina V FITC,* Abcam, Cambridge, UK), following the instructions for use provided by the producer. All samples were analyzed with a *FACSCalibur* flow cytometer equipped with an argon laser at 488 nm (Becton Dickinson). The data thus obtained were analyzed with *Cell Quest* software. The apoptosis percentage was calculated by considering the cells as being in early (Annexin + PI-) and late (Annexin + PI+) apoptosis.

### Effect of the aggregated phospholipid-based nanovesicles incorporating curcumin on the IL-10 inflammation factor

The synoviocytes obtained from patients suffering from rheumatoid arthritis, and maintained in culture in the presence of synovial liquid for 5 days, were treated with curcumin in dispersion or incorporated into hyalurosomes or into aggregated phospholipid-based vesicles (Form A). Subsequently, they were fixed, made permeable and incubated with monoclonal antibodies (anti-IL-10). After about 2 hours the cells were washed with PBS and Tween-20 and incubated for 1 hour with secondary antibodies. Cells treated with secondary antibodies only were used as a negative control. After this treatment, the absorbance of the solutions contained in the 96-well plates was read by using an *Odyssey Infrared Imaging System* apparatus (*LI-COR,* Bioscence) in order to measure the existing cytokins.

### 1.6 In vitro biocompatibility of the aggregated phospholipid-based vesicles carrying the molecules under examination

### Culture of keratinocytes and fibroblasts

Human keratinocytes were isolated from the skin of healthy volunteers. The cutaneous biopsies were separated from the subcutaneous tissue and from the dermis with the help of sterile scalpels and then cut into fragments of approx. 1 mm², which were made to adhere to the bottom of the plates and incubated with the culture medium. The cells were grown as monolayers in 35mm plates, incubated at 37°C with 100% humidity and 5% CO₂. RPMI 1640 medium was used as a culture medium, enriched with L-Glutamine, fetal bovine serum (20%), penicillin/streptomycin (1%) and fungizone (1%). The culture medium was changed every two days to ensure cell growth and to avoid any contamination. Sterility was ensured by using sterile media and materials and by manipulation in a controlled environment (laminar flow hood). Once sub-confluence had been achieved, the cells were detached with trypsin (0.25%) from the bottom of the plate, centrifuged (1,600 rpm, 4 min), and then re-suspended in fresh culture medium at a concentration of 7.5 × 10³ cells/plate.

Mouse embryonic fibroblasts (3T3) (ATCC collection, United States of America) were used. In this case as well, the cells were grown as monolayers in 35mm plates, incubated at 37°C with 100% humidity and 5% CO₂. DMEM was used as a culture medium, enriched with fetal bovine serum (20%), penicillin/streptomycin (1%) and fungizone (1%). The culture medium was changed every two days to ensure cell growth and to avoid any contamination. Once sub-confluence has been achieved, the cells were detached with trypsin (0,25%) from the bottom of the plate, centrifuged (1,600 rpm, 4 min), and then re-suspended in fresh culture medium at a concentration of 7.5 × 10³ cells/plate.

### Measurement of cell viability by MTT assay

The effect of the aggregated phospholipid-based nanovesicles on dermal or epidermal cells was studied by measuring cell viability with the tetrazolium salt colorimetric assay, 3-(4,5-dimethylthiazol-2-yl)-2,5-diphenyltetrazolium bromide, (MTT). By estimating the number of viable cells in a culture, it is possible to evaluate the effect of the treatment with the formulations under examination on the viability of the cell population. The MTT compound is reduced by a mitochondrial enzyme, succinate dehydrogenase, with formation of formazan, a water-insoluble compound that forms blue-violet crystals. Unlike non-viable cells, viable cells reduce MTT, and the amount of produced formazan is proportional to the number of existing viable cells. In the course of our study, the formed crystals were solubilized in dimethyl sulfoxide and the absorbance values were measured at 570 nm by using a *Synergy 4, Synergy^{™} Multi-Detection Microplate Reader* (BioTek Instruments, AHSI S.P.A, Bernareggio, Italy). The cells (keratinocytes or fibroblasts, 7.5 × 10³ cells/well) were sown in 96-well plates with 250 µl of culture medium. After sowing (16 hours), the cells were treated for 24 and/or 48 hours with the aggregated phospholipid-based nanovesicles of the present invention and/or with the comparison vesicles, and/or with the dispersion of the substances under examination. For each sample, the study was repeated three times. Untreated cells were used as a control. Cell viability at the end of the experiment was calculated as a percentage relative to untreated cells (negative control indicating 100% viability).

### 1.7 Effect of the aggregated phospholipid-based nanovesicles carrying baicalin on the viability of cells stressed with hydrogen peroxide

The protective effect exerted by the aggregated phospholipid-based nanovesicles on the viability of cells subjected to oxidative stress with hydrogen peroxide was measured. The cells were sown in 96-well plates and incubated at 37°C with 5% CO₂ for the period necessary for achieving confluence. As a negative control (100% viability), untreated cells cultivated in optimal growth conditions were used. As a positive control, cells treated with hydrogen peroxide only were used, without the samples containing the three molecules under examination. As confluence was achieved, the cells were treated with hydrogen peroxide and at the same time also with the samples containing the different molecules under examination, whether in dispersion or in vesicular formulation form. The cells were incubated for 4 hours, washed with PBS and treated with MTT in order to measure their survival.

### 1.8 Anti-microbial sensitivity test on the aggregated phospholipid-based nanovesicles carrying clotrimazole

### Strains used

Anti-microbial activity was tested by using some pathogenic agents of human skin: *Candida albicans* (OG11) and *Candida krusei* (BF2). The yeasts were cultivated in *Sabouraud agar* medium at 37°C. The formulations of clotrimazole or its dispersion in ethanol, glycerol and water (100 µl) were suspended in Petri plates in the presence of 100 µl of microbial inoculum. Each strain was inoculated in exponential phase and, after 24 hours of growth at 37°C, the inhibition halo was measured. Each experiment was repeated 3 times.

### 1.9 In vivo study of edema and myeloperoxidase inhibition given by the aggregated phospholipid-based nanovesicles carrying the molecules under examination

For *in vivo* experiments, Hsd: ICR (CD-1^{®}) female mice 5-6 weeks old (weight 25-35 g) bought from Harlan Laboratories (Barcelona, Spain) were used. The mice were left to acclimatize for 1 week prior to use. All studies were conducted in compliance with the European Union regulations concerning management and use of laboratory animals. The protocols had been approved by the Institutional Committee for the care and use of animals of the University of Valencia. The back of the mice was shaved (~2 cm²) and 12-O-tetradecanoylphorbol-13-acetate (TPA) dissolved in acetone (3 µg/20 µl) was applied thereon to induce cutaneous inflammation and ulceration (day 1). On the mice used as positive controls only acetone (20 µl) was applied. 3 hours after the application of TPA, the dispersions of baicalin carried in aggregated vesicles or in transfersomes were applied (40 µl) drop by drop. The procedure was repeated (at 24-hour intervals) on days 2 and 3. On day 4, the mice were sacrificed by cervical dislocation. Each group was composed of four mice. Inflammation and ulcer inhibition was quantified by measuring the formation of edema and the concentration of myeloperoxidase (MPO).

The treated dorsal skin area was removed and weighed in order to evaluate any increase in weight indicative of edema formation. For the MPO measurement, the cutaneous biopsies were homogenized and centrifuged, and then the surnatant was incubated with hydrogen peroxide and tetramethyl benzidine. MPO activity was quantified by spectroscopy at 620 nm. The ulceration degree was evaluated visually.

### Examples of Preparation of Aggregated Nanovesicles

### EXAMPLE 1: Preparation of aggregated phospholipid-based nanovesicles according to the invention containing clotrimazole, chemical-physical characterization and evaluation of their anti-mycotic activity

Clotrimazole, a synthetic anti-microbial agent, was incorporated into the aggregated phospholipid-based nanovesicles by using lecithin as a phospholipid (Galeno, Prato, Italy) and, as a scattering medium, 4 different mixtures of aqueous co-solvents obtained by using different concentrations of ethanol, glycerol and water. In confirmation of the good loading capacity of the system, it was possible to load into the aggregated phospholipid-based nanovesicles 10 mg/ml of clotrimazole, while it was not possible to load the same quantity of active agent into the conventional liposomes used as a reference. The capability of the aggregated vesicles to promote dermal and transdermal penetration of the drug was measured *in vitro* by using newborn pig skin and Franz diffusion cells. As comparisons, the dispersion of clotrimazole in ethanol, glycerol and water and a commercial formulation containing the same quantity of active principle (Canesten^{®}) were used.

The *in vitro* anti-microbial activity of the different formulations was also measured, and in this study as well it was compared with the one of Canesten^{®} and of the dispersion of clotrimazole (10 mg/ml) in ethanol, glycerol and water. The composition of the vesicles is shown in the following Table 1.

**Table 1. - Composition of the different formulations of aggregated phospholipid-based vesicles incorporating clotrimazole**

| | Lecithin (mg/ml) | Clotrimazole (mg/ml) | Ethanol (ml) | Glycerol (ml) | Water (ml) |
|---|---|---|---|---|---|
| Form A | 180 | 10 | 39 | 59 | 2 |
| Form B | 180 | 10 | 36 | 54 | 10 |
| Form C | 180 | 10 | 29 | 69 | 2 |
| Form D | 180 | 10 | 27 | 63 | 10 |

### EXAMPLE 2: Preparation of aggregated phospholipid-based nanovesicles according to the invention incorporating curcumin, chemical-physical characterization and evaluation of the in vitro anti-inflammatory effectiveness thereof

The vesicles were prepared by using a commercial mixture of phospholipids, containing mainly soy phosphatidylcholine (trade name P90G, supplied by AVG S.r.l., Garbagnate Milanese, Italy on behalf of Lipoid GmbH, Ludwigshafen, Germany). Curcumin (10 mg/ml) was incorporated into the prepared aggregated nanovesicles with 180 mg/ml of phospholipids and a mixture of ethanol, glycerol and water (29/69/2 v/v of the carrier) as a dispersing carrier, the composition of which is shown in the following Table 2. Conventional liposomes prepared with the same quantity of phospholipid and curcumin are unstable and precipitate shortly afterwards; therefore, curcumin hyalurosomes and a rough dispersion of curcumin in ethanol, glycerol and water, prepared extemporaneously, were used as comparisons. Hyalurosomes are vesicles of phospholipids and sodium hyaluronate that have showed very good results in cutaneous application of curcumin (Manca et al. 2014); in this case, they were prepared with the same quantity of phospholipid and curcumin and a solution of sodium hyaluronate at 0.2% (w/v). The study also evaluated the capability of the vesicular formulations of the present invention to promote the *in vitro* anti-inflammatory effectiveness of curcumin by using synoviocytes in the presence of synovial liquid.

**Table 2. - Composition of the aggregated phospholipid-based nanovesicles incorporating curcumin.**

| | P90G (mg/ml) | Curcumin (mg/ml) | Ethanol (ml) | Glycerol (ml) | Water (ml) |
|---|---|---|---|---|---|
| Form A | 180 | 10 | 29 | 69 | 2 |

### EXAMPLE 3: Preparation of aggregated phospholipid-based nanovesicles according to the invention and carrying baicalin, chemical-physical characterization, evaluation of the capability of the same of promoting accumulation of baicalin in the skin and its in vitro and in vivo anti-inflammatory and anti-oxidant effectiveness

Vesicles carrying baicalin (10 mg/ml) were prepared by using a commercial mixture of phospholipids, containing mainly soy phosphatidylcholine (trade name S75, supplied by AVG S.r.l., Garbagnate Milanese, Italy on behalf of Lipoid GmbH, Ludwigshafen, Germany). Three different formulations were prepared, containing 180 mg/ml of S75 and, as a dispersing carrier, three different mixtures of ethanol, glycerol or propylene glycol and water, the composition of which is shown in the following Table 3. Since it was impossible to prepare a stable liposome preparation by using the same quantities of phospholipids, baicalin and water, transfersomes obtained by adding Tween 80 (2.5 mg/ml) to the phospholipid and by hydration with a dispersion of baicalin in phosphate buffer at pH 7.4 (PBS) were used as a comparison. The nanovesicles capability of promoting dermal and transdermal penetration of baicalin was studied *in vitro* by using newborn pig skin and Franz diffusion cells. The study also evaluated the *in vitro* biocompatibility of the formulations by using keratinocytes and fibroblasts, the *in vitro* anti-oxidant effectiveness with the same cells, and the *in vivo* anti-inflammatory effectiveness in a model of cutaneous inflammation.

**Table 3. Composition of the preparations of aggregated phospholipid-based nanovesicles of the invention carrying baicalin.**

| | S75 (mg/ml) | Baicalin (mg/ml) | Ethanol (ml) | Glycerol (ml) | PG (ml) | Water (ml) |
|---|---|---|---|---|---|---|
| Form A | 180 | 10 | 30 | 34 | 34 | 2 |
| Form B | 180 | 10 | 30 | 0 | 68 | 2 |
| Form C | 180 | 10 | 30 | 68 | 0 | 2 |

### Experimental results of the above preparation examples

### RESULTS OF EXAMPLE 1: Clotrimazole

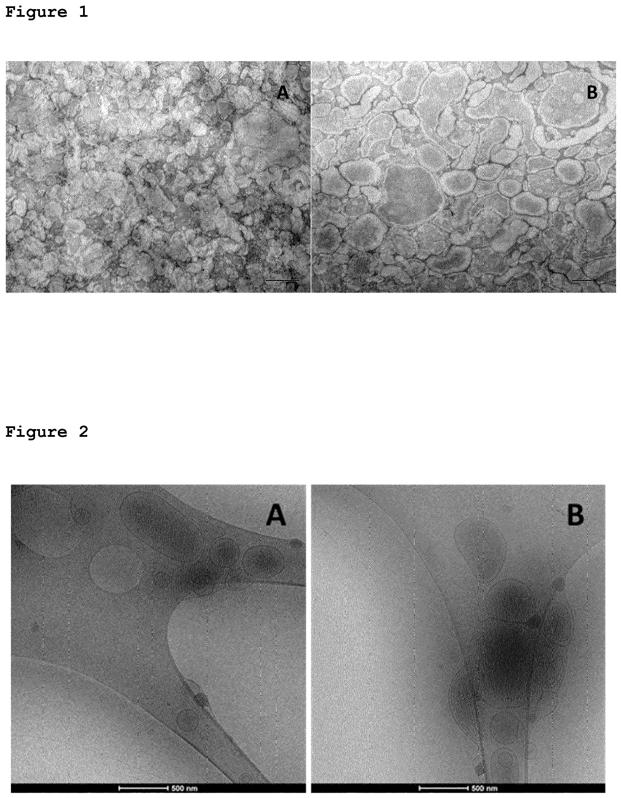

Clotrimazole was stably incorporated into the aggregated phospholipid-based nanovesicles of the present invention, but it was not possible to incorporate it at the same concentration into conventional liposomes or into other liposome-like vesicles. In fact, after preparation, formation of a precipitate on the bottom of the test tube was always observed, as a proof of their instability. The formation of three-dimensionally aggregated nanovesicular structures was confirmed under transmission electron microscope (TEM), as can be seen in **Figure** 1. The vesicles have a quite regular spherical shape. Multi- and uni-lamellar structures coexist in the samples, and the vesicles are mostly aggregated with one another, forming a three-dimensional network. Following dilution of the samples in water (1/500), it was possible to freeze the vesicles and observe them with cryo-TEM. In these conditions, the aggregated nanovesicles of the present invention become single lamellar vesicles, like conventional liposomes. In this case as well, these are single multi- and uni-lamellar structures coexisting with one another, see **Figure 2****.**

Table 4 below indicates the mean diameter, polydispersity index (PI), zeta potential and incorporation efficiency (E) of the aggregated phospholipid-based vesicles prepared in Example 1 and carrying clotrimazole. The mean values ± standard deviation obtained by measuring at least six samples (n=6) are indicated. Small aggregated nanovesicles were obtained, having a mean diameter never exceeding 200 nm, quite homogeneous dimensions (PI ≤0.3) and high incorporation efficiency (E) exceeding 80% (Table 4). In particular, formulations A and C, which contain less water (2% v/v of the dispersing carrier) have smaller dimensions than those (B and D) containing more water (10% v/v of the dispersing carrier). The conventional liposomes obtained by using water only as a hydration solvent have greater dimensions (approx. 350 nm), the highest polydispersity index (approx. 0.5) and, most importantly, after 2-3 hours the drug precipitates, indicating the inability of these vesicles to completely incorporate it into their bilayer. Due to the instability of the liposome dispersion, it was not possible to characterize it or test its *in vitro* properties.

**Table 4**

| | Mean diameter (nm) | PI | Zeta potential (mV) | E (%) |
|---|---|---|---|---|
| Form A | 96 ± 2 | 0.26 | -64 ± 58 | 95 ± 5 |
| Form B | 186 ± 6 | 0.29 | -59 ± 2 | 86 ± 5 |
| Form C | 129 ± 7 | 0.28 | -77 ± 1 | 98 ± 8 |
| Form D | 140 ± 11 | 0.23 | -61 ±1 | 81 ± 7 |

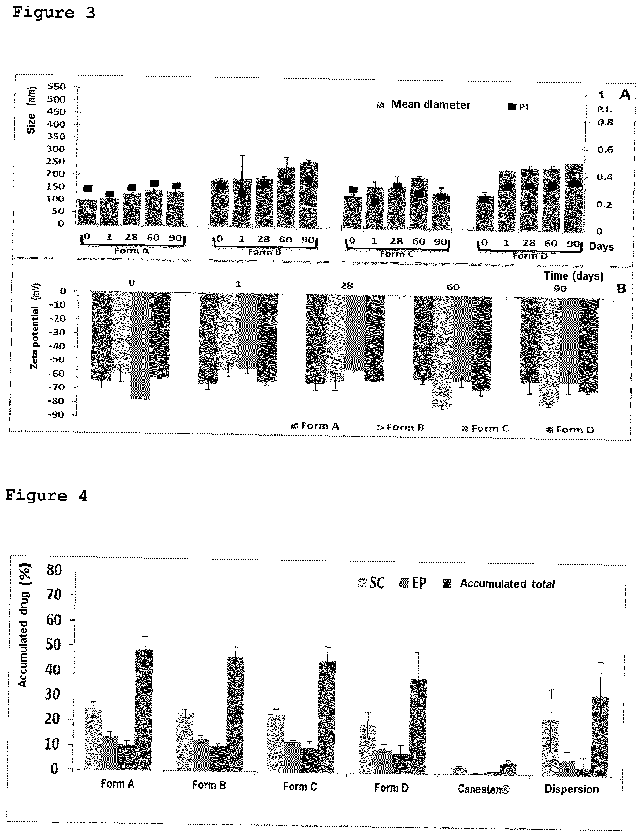

In order to evaluate the stability of the formulations over time, the samples were preserved at 25°C exposed to light for 90 days, and the respective values of mean diameter, polydispersity index and zeta potential were measured on the day of preparation and after 30, 60, 90 days (**Figure 3**). During the 90 days of the study, no formation of precipitate was observed in any sample, and the measured values indicate that all formulations are stable over time, especially those containing less water (2% v/v of the dispersing carrier). In fact, the dimensions of the nanovesicles of these latter formulations (A and C) and their dimensional distribution (PI ≤0.3) remained constant, whereas after 90 days those of the two formulations containing more water (10% v/v of the dispersing carrier) had grown by 41% (sample B) and 92% (sample D), and their polydispersity index had increased (**Figure 3**).

*In vitro* cutaneous permeation studies, conducted by using Franz diffusion cells and newborn pig skin, confirmed the better capability of the aggregated phospholipid-based nanovesicles of promoting accumulation of clotrimazole in the corneous layer, and especially in the epidermis and in the dermis, compared to the dispersion in the mixture of solvents of the present invention and the commercial formulation containing the same quantity of active principle (Canesten^{®}), (**Figure 4**).

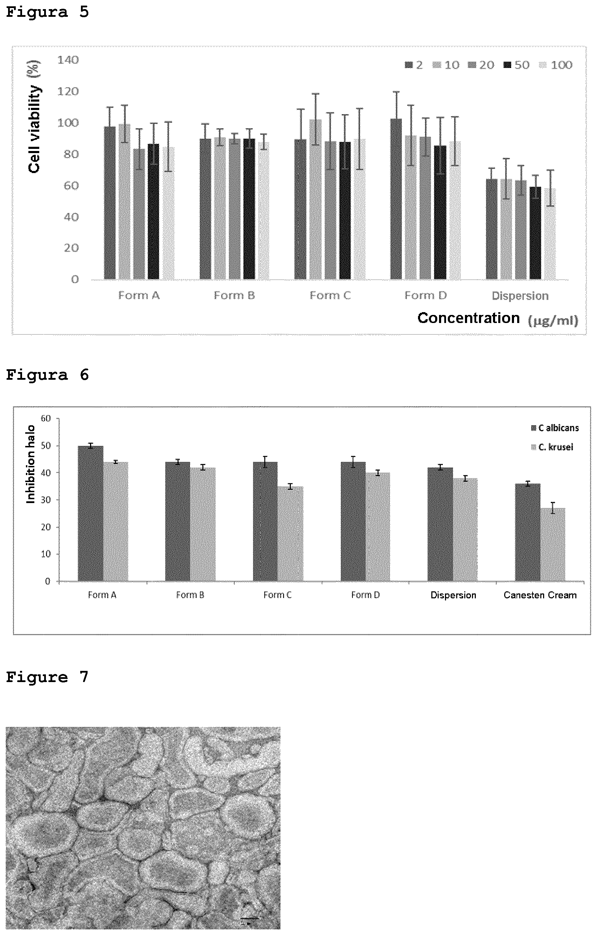

The *in vitro* biocompatibility of the aggregated phospholipid-based nanovesicles incorporating clotrimazole was studied by using keratinocytes, which are the cells that constitute most of the epidermis (**Figure 5**). Keratinocytes were incubated for 48 hours by using 5 different concentrations of each sample (nanovesicles incorporating clotrimazole and its dispersion). At the end of the experiment, cell viability was measured by MTT assay. As can be observed in **Figure 4****,** the aggregated phospholipid-based nanovesicles have no significant toxicity at all concentrations used; in fact, viability is always greater than 80%. The clotrimazole dispersion causes higher mortality, equal to ~40% (viability ~60%), also at higher dilutions.

The ability of the aggregated phospholipid-based nanovesicles incorporating clotrimazole to inhibit the growth of candida, a saprophytic fungus belonging to the family of Saccharomycetes, was assessed. The anti-mycotic power of the aggregated phospholipid-based nanovesicles incorporating clotrimazole was compared with that of the dispersion of clotrimazole in the mixture of solvents of the present invention and that of the commercial formulation Canesten^{®}. As can be observed in **Figure 5**, the clotrimazole carried in the aggregated nanovesicles of the present invention inhibits the proliferation of both candida strains in use to a considerably greater extent than the commercial formulation.

### RESULTS OF EXAMPLE 2: Curcumin

Curcumin was carried in the aggregated phospholipid-based nanovesicles of the present invention in order to improve its local bioavailability in articulations, whereon it is supposed to exert its effect in patients suffering from rheumatoid arthritis by promoting the production of IL-10 and reducing the proliferation of synoviocytes, which are connective cells with peculiar characteristics that coat the inside of the synovial membranes. The results could not be compared with those of conventional liposomes because, in this case as well, the formulation is unstable and the drug precipitates. Curcumin hyalurosomes were thus prepared, which in a previous study had shown very good performance in promoting the anti-inflammatory effectiveness of curcumin in cutaneous wounds. Hyalurosomes were prepared with the same quantities of phospholipid, but a dispersion of curcumin and sodium hyaluronate at 0.2% (w/v) in water was used as a dispersing carrier. As a comparison, also the dispersion of curcumin in ethanol, glycerol and water (29/69/2 v/v) was used, prepared extemporaneously. The actual formation of nanovesicular structures and the morphology thereof was confirmed under transmission electron microscope; as can be seen in **Figure 7**, oligo-lamellar vesicles are formed, wherein multi- and uni-lamellar vesicles coexist, which have an irregular shape and are tightly interconnected.

The aggregated phospholipid-based nanovesicles prepared with ethanol, glycerol and water have a mean diameter of ~161 nm and a polydispersity index of approx. 0.23, which indicates good system homogeneity. The hyalurosomes have a slightly greater mean diameter (∼180 nm) and polydispersity index (~0.26). Further additions of water or ethanol to the scattering carrier destabilize the system and cause vesicle precipitation, so that it was not possible to obtain stable formulations with different proportions of ethanol, glycerol and water. As regards the nanovesicles of formulation A, the zeta potential value is close to neutrality (~-3 mV), due to the lower dielectric constant of the dispersing carrier compared to that of water. This facilitates the approaching of the vesicles and the formation of the three-dimensional structure. The potential of hyalurosomes is considerably more negative (∼-30 mV) and allows for electrostatic stabilization of the single vesicles in dispersion. The aggregated nanovesicles of the present invention incorporate a large quantity of curcumin (E ~88%), equal to that carried by the hyalurosomes studied as a specific system for delivery of curcumin, because hyaluronic acid is able to scatter it in a stable manner (Table 5).

**Table 5**

| | Mean diameter (nm) | Polydispersity index PI | Zeta potential (mV) | E (%) |
|---|---|---|---|---|
| Form A | 161 ± 16 | 0.23 | -3 ± 1 | 88 ± 7 |
| Hyalurosomes | 180 ± 11 | 0.26 | -30 ± 7 | 88 ± 6 |

Both formulations are sufficiently stable. In particular, the dimensions of the hyalurosomes remained virtually unchanged during the study period, whereas those of the aggregated nanovesicles showed a slight increase (~30%), as can be observed in **Figure** 8. Anyway, both formulations, although their dimensions change, did not release the curcumin; as a matter of fact, their incorporation efficiency remained constant during the study period **(****Figure** 8).

Pro-apoptotic capacity and production of IL-10 induced by curcumin (incorporated into aggregated vesicles or hyalurosomes or dispersion) was studied on synoviocytes obtained from patients suffering from rheumatoid arthritis and maintained in culture by using a medium enriched with synovial liquid to re-create *in vitro* the *in vivo* conditions. As can be observed in **Figure 9****,** the aggregated nanovesicles of formulation A can induce cell death to a significantly greater extent than the hyalurosomes and the curcumin dispersion used as a comparison. It can thus be assumed that the formulations are able to reduce synovial cell proliferation *in vivo,* avoiding the lymphocyte and macrophage infiltrations that are typical of the articular inflammation caused by rheumatoid arthritis. Moreover, the aggregated nanovesicles incorporating curcumin stimulate the production of IL-10 to a significantly greater extent than the hyalurosomes and the dispersion in ethanol, glycerol and water **(****Figure** 10). In fact, the intense cell hyperplasia and proliferation that occur in the synovial liquid in patients suffering from rheumatoid arthritis is compensated for, in our organism, by an anti-inflammatory response characterized by the production of antagonists of IL-1 and IL-10. In particular, the levels of IL-10 are generally high in the blood and synovial liquid of patients suffering from rheumatoid arthritis.

### RESULTS OF EXAMPLE 3: Baicalin

Table 6 below indicates the mean diameter, polydispersity index (PI), zeta potential and loading efficiency (E) of the nanovesicles of the present invention carrying baicalin. The mean values ± standard deviation (n=6) are indicated.

**Table 6**

| Samples | Diameter (nm) | PI | Zeta potential (mV) | E (%) |
|---|---|---|---|---|
| Transfersomes | 79±6 | 0.21 | -51±6 | 37±18 |
| Form A | 60±12 | 0.37 | 0±2 | 68±15 |
| Form B | 38±13 | 0.47 | 0±1 | 69±5 |
| Form C | 174±17 | 0.33 | 1±3 | 20±5 |

Baicalin was carried in three different formulations of aggregated phospholipid-based nanovesicles by using, as a scattering carrier, three mixtures of ethanol, glycerol and/or propylene glycol and water. Transfersomes containing 2.5 mg/ml of Tween 80 and hydrated with a dispersion of baicalin in phosphate buffer at pH 7.4 were prepared as a comparison. These are elastic vesicles known for their ability to pass intact through the corneous layer, thus promoting dermal accumulation of the carried substances. The tranfersomes have very small dimensions (~79 nm), a low polydispersity index (0.21), and a highly negative potential. The vesicles of formulation A are small, ~64 nm, those of formulation B are even smaller, ~38 nm, while those of formulation C are much bigger than the transfersomes and the other aggregated vesicles. In this case, the increased quantity of propylene glycol causes a rearrangement of the bilayer, resulting in an increased curvature of the membrane and originating bigger, and most likely less lamellar (see TEM), vesicles (Castangia et al. 2014). The polydispersity index is slightly high (0.33-0.47) for all dispersions: this indicates that the samples are partially polydispersed. Anyway, the dimensions are always repeatable, as confirmed by the low standard deviation values obtained in at least six repetitions (Haidar et al. 2008). The potential of all samples is virtually null (~0 mV), probably because of the lower dielectric constant of the dispersing medium compared to water.

The vesicles' ability to carry baicalin, expressed as loading efficiency (E), was calculated as a percentage ratio between the baicalin concentration in the dispersion before and after the dialysis (El Azim et al. 2015). In fact, this process allows eliminating the molecules that are not being effectively carried in the vesicles. The efficiency of formulations A and B (~65%) is higher than that of transfersomes, whereas the efficiency of formulation C is much lower, probably because propylene glycol has a strong solubilizing effect and, in addition to modifying the bilayer, also increases the solubility of baicalin and hence decreases the quantity thereof that remains intercalated in the lipid bilayer.

The formulations were preserved at 5°C away from light for a period of 12 months, during which the size, polydispersity index and zeta potential were measured (**Figure 12**). The study highlighted that all the aggregated vesicles are as stable as the transfersomes used as a reference, and the characteristics of the vesicles remain unchanged (±10%) throughout the period. The zeta potential values remain constant (~0 mV) throughout the study period (values not shown).

When tranfersomes carrying baicalin are applied, ~2% of the quantity of phytodrug reaches the corneous layer, ~1% the epidermis, ~5% the dermis (the greatest amount), and ~0.5% the receptor compartment, with prevalent accumulation in the dermis **(****Figure** 13). When baicalin is carried in formulations B and C of aggregated vesicles, the total quantity deposited in the skin (~7%) is comparable to that which is found when transfersomes are used. Unlike transfersomes, all formulations of aggregated vesicles increase the quantity of baicalin permeating into the receptor compartment, which amounts to ~4% for formulation C, ~6% for formulation B, and ~8% for formulation A. Therefore, thanks to their ability as carriers, the aggregated vesicles of the present invention allow for simultaneous *in vitro* permeation of baicalin into the receptor compartment and accumulation thereof in the dermis (≈6%), and presumably also in subcutaneous tissues *in vivo.* The very good performance of these vesicles is due to the combination of phospholipids with different alcoholic aqueous solvents that do not destroy the vesicular structure and help them reach the dermis (Mir-Palomo et al., 2016). The possible toxic effects of the formulations were evaluated *in vitro* by using the main dermal and epidermal cells, more specifically human keratinocytes and a line of mouse embryonic fibroblasts (3T3). The cells were incubated by adding the four different baicalin formulations, at increasing dilutions, to the culture medium, and after 48 hours **(****Figure 14****)** cell survival was measured with reference to that of untreated cells (100% viability). When using transfersomes, the viability of keratinocytes and fibroblasts is ~100% at all dilutions; therefore, their toxicity is null. The viability of keratinocytes incubated with the formulations of aggregated nanovesicles is slightly lower, but still greater than 90%, at the lowest dilutions (20-100 µg/ml of baicalin), and grows even higher and in excess of 100% when using the highest dilutions (2 and 10 µg/ml). The viability of fibroblasts is always ≥100%, regardless of the formulation and dilution used. These results suggest that all formulations are safe and biocompatible with the cells of the skin.

Taking into account the well-known antioxidant and protective properties of baicalin, the ability of the formulations to protect keratinocytes and fibroblasts from oxidative stress caused by hydrogen peroxide was evaluated. The cells were stressed with hydrogen peroxide while being treated with baicalin (5 µg/ml) carried in transfersomes and in the formulations of aggregated vesicles. One group of cells was not treated either with hydrogen peroxide or with the formulations, and was used as a negative control (100% viability); another group was treated with hydrogen peroxide only (positive control). After 4 hours of incubation, in the positive control group of keratinocytes hydrogen peroxide causes a mortality of 40% (60% viability relative to the negative control), whereas in the fibroblast group mortality is lower, i.e. 30% (70% survival). The treatment with transfersomes leads to increased viability of keratinocytes (95%) and, to a lesser extent, fibroblasts (80%) **(****Figure** 15). The treatment with the formulations of aggregated vesicles results in increased viability, approx. 100% for keratinocytes and in excess of 100% for fibroblasts, independently of the composition of the formulations (Ron-Doitch et al. 2016). The results of the test of cell survival to oxidative stress highlight a good ability of all the formulations of aggregated vesicles carrying baicalin, better than that of the comparison transfersomes, to effectively counter the detrimental effects of free radicals.

*In vivo* studies on the anti-inflammatory effectiveness of baicalin, carried in the aggregated phospholipid-based nanovesicles of the present invention, were conducted on mice with back skin inflammation induced by local application of TPA, a highly irritating substance that, when applied 2-3 times in a row on the skin, produces inflammation with edema formation, neutrophil infiltration and increased myeloperoxidase (MPO), which is an enzyme indicative of the inflammatory state **(****Figure 16**). Application of baicalin carried in transfersomes causes a strong inhibition of the edema (~55%) and a low inhibition of MPO (~20%), whereas the effect of baicalin carried in the aggregated vesicles is opposite, in that edema inhibition is low (~30%) and MPO inhibition is much higher (~75%). This might be due to the specific dermal penetration capability of the different vesicles: transfersomes cause preponderant accumulation of baicalin in the dermis and are more effective in inhibiting the edema, while the aggregated vesicles promote subcutaneous accumulation and are more effective in inhibiting MPO.

### Advantages and Industrial Applicability of the Invention

The formulation of aggregated nanovesicles of the present invention, as previously described and set out in the appended claims, allowed overcoming or reducing the limitations encountered when using conventional liposomes and liposome-like vesicles (loading high concentrations of active agents, stability in dispersion, and ability to promote the passage of active agents through biological membranes, especially the skin) due to the fact that they were prepared by using, as a dispersing carrier, a mixture formed by multiple water-miscible solvents with less or no water (0-25%) in said mixture. It was thus possible to prepare aggregated lamellar phospholipid-based nanovesicles capable of maintaining their own structure or morphology even after high dilutions in water (1/500 v/v) or saline (which mimics biological liquids). Furthermore, the vesicles of the present invention are characterized by high stability over time, so that they will retain their initial characteristics and remain in dispersion. The use of a mixture of water-miscible solvents (defined as aqueous solvents in the present document) for hydrating the phospholipids, instead of water as used in the liposomes taken into account as a reference, allowed increasing the quantity of active substance, especially lipophilic ones, solubilized in the dispersing medium, and hence the loading capacity of the nanovesicles. At the same time, the aqueous co-solvents have a lower dielectric constant than water and lead to reduced surface charge of the nanovesicles and mutual repulsion force, and cause a variation in the orientation of the phospholipid chains, in particular of the hydrophobic tails, some of which face towards the external phase, interacting with the other vesicles to form a three-dimensional supravesicular structure/aggregate that makes their dispersions particularly stable (Castangia et al., 2015). When diluted in biological liquids, these nanovesicles lose their three-dimensional structure and disperse as single nanovesicles, thus behaving like liposomes, so that they are stable at high dilutions and can interact with the biological membranes, also because of their high concentration of alcohols and polyalcohols, which behave as absorption promoters. These aggregated nanovesicles can solubilize, in a water-miscible carrier, high concentrations of drugs or active molecules of natural and/or synthetic origin, even those poorly water-soluble, and can also keep the carried active agent for a long time within the nanovesicular structure, due to their high stability that allows them to keep their own structure unchanged over time, without breaking or fusing and releasing the molecules being carried. The aggregated vesicles of the present invention proved to be able to solubilize and retain within their own structure also high concentrations of highly lipophilic active molecules, which the other liposome-like phospholipid-based vesicles are not able to carry, at least at the same concentration.

These aggregated phospholipid-based vesicles of the present invention allowed dispersing hydrophilic and lipophilic active molecules (much more preferably, lipophilic ones) in a water-miscible carrier, thus making them bioavailable for absorption through biological membranes (in particular, the skin). This absorption promoting effect was confirmed by enhanced dermal and transdermal absorption of several lipophilic active substances. For example, the *in vitro* studies confirmed that these formulations of the invention can increase, also compared to traditional liposomes or transfersomes, the quantity of active agent accumulating in the skin and passing through it. The *in vivo* studies conducted on animal models confirmed a considerable enhancement of therapeutic effectiveness on animal models, which showed a remarkable improvement in the therapeutic effectiveness of some pharmacologically or biologically active molecules when carried in aggregated phospholipid-based nanovesicles dispersed in a carrier of alcohols and/or polyalcohols according to the present invention. All limitations of the other liposome-like vesicles have been overcome by the nanovesicles of the present invention.

In addition to being stable, these formulations are also very viscous; therefore, application on the skin is easier because the formulation remains on the site of application for a longer time without slipping away. At the same time, the nanovesicles of the invention are diluted in contact with the endogenous water of the skin and modify their own aggregation state, thus losing the three-dimensional structure and dispersing as single vesicles that can easily interact with the skin surface. After being diluted in aqueous liquids, they behave like single liposomal vesicles, with the difference that within their structure they mostly contain water-miscible solvents that are less polar than water, like ethanol, which behaves as an absorption promoter and facilitates the passage through the skin; glycerol behaves as a hydrating agent and facilitates the relaxation of the cutaneous matrix; other polyalcohols behave as hydrating agents and absorption promoters at the same time.

The aggregated nanovesicular structure taken by the phospholipids of the present invention allows achieving the following additional advantages:
- increased capacity of loading and/or carrying lipophilic active agents of natural or synthetic origin, compared to known vesicular systems, because, the phospholipid in use being the same, the aggregated nanovesicles have an almost continuous packing structure, which allows it to trap the active substances not only within the vesicles, but also in the inter-vesicular three-dimensional structure. Moreover, the aqueous solvents employed generally have higher solvent power than water, especially towards substances having a lipophilic character;
- increased stability compared to known liposomal systems, which over time tend to aggregate and fuse, thus releasing the incorporated active agents; the nanovesicles of the present invention are very small and structured, so that they will not tend to precipitate and fuse;

- increased capability of retaining the active agents for a longer time, because the latter are stably dissolved at molecular level into the co-solvents and/or between the lipophilic chains of the phospholipids;
- increased capability of passing through the skin, compared to the other topical release systems of vesicular nature, due to the synergic action of the phospholipids and of the carrier composed of alcohols and/or polyalcohols, which behave as absorption promoters;
- increased solubilization of the lipophilic active agents being carried, with an enhancement of their kinetics of dissolution in biological fluids, resulting in better bioavailability of the active agents on the site of action.

### List of bibliographic references

Castangia, I., Manca, M.L., Matricardi, P., Catalán-Latorre, A., Nácher, A., Diez-Sales, O., Fernàndez-Busquets, X., Fadda, A.M., Manconi, M., 2015. Effects of ethanol and diclofenac on the organization of hydrogenated phosphatidylcholine bilayer vesicles and their ability as skin carriers. J. Mater. Sci. Mater. Med. 26, 137. doi:10.1007/s10856-015-5443-1
Castangia, I., Nácher, A., Caddeo, C., Valenti, D., Fadda, A.M., Díez-Sales, O., Ruiz-Saurí, A., Manconi, M., 2014. Fabrication of quercetin and curcumin bionanovesicles for the prevention and rapid regeneration of full-thickness skin defects on mice. Acta Biomater. 10, 1292-1300. doi:10.1016/j.actbio.2013.11.005
Haidar, Z.S., Hamdy, R.C., Tabrizian, M., 2008. Protein release kinetics for core-shell hybrid nanoparticles based on the layer-by-layer assembly of alginate and chitosan on liposomes. Biomaterials 29, 1207-15. doi:10.1016/j.biomaterials.2007.11.012
Manca, M.L., Manconi, M., Nacher, A., Carbone, C., Valenti, D., Maccioni, A.M., Sinico, C., Fadda, A.M., 2014. Development of novel diolein-niosomes for cutaneous delivery of tretinoin: influence of formulation and in vitro assessment. Int. J. Pharm. 477, 176-86. doi:10.1016/j.ijpharm.2014.10.031
Mir-Palomo, S., Nácher, A., Díez-Sales, O., Busó, M.A.O.V., Caddeo, C., Manca, M.L., Manconi, M., Fadda, A.M., Saurí, A.R., 2016. Inhibition of skin inflammation by baicalin ultradeformable vesicles. Int. J. Pharm. doi:10.1016/j.ijpharm.2016.06.136
Ron-Doitch, S., Sawodny, B., Kühbacher, A., David, M.M.N., Samanta, A., Phopase, J., Burger-Kentischer, A., Griffith, M., Golomb, G., Rupp, S., 2016. Reduced cytotoxicity and enhanced bioactivity of cationic antimicrobial peptides liposomes in cell cultures and 3D epidermis model against HSV. J. Control. Release 229, 163-171. doi:10.1016/j.jconrel.2016.03.025

## Claims

1. Three-dimensionally aggregated, phospholipid-based nanometric vesicles dispersed in an alcoholic mixture, comprising or consisting of:
a) at least one phospholipid selected among one or more natural or synthetic phospholipids, whether pure or blended, selected from the group including soy or egg lecithin, phosphatidylcholine at different degrees of purity, whether hydrogenated or non-hydrogenated, phosphatidylethanolamine, phosphatidylserine, phosphatidylglycero1, phosphatidylinosito1, dimyristoylphosphatidylcholine, dipalmitoylphosphatidylcholine, distearoylphosphatidylcholine, sphyngomyelin, palmitoylstearoylphosphatidylcholine, hydrogenated and non-hydrogenated phospholipids derived from soy and/or mixtures thereof;
b) at least one pharmaceutical and/or cosmetic active principle, one biologically active molecule of natural or synthetic origin, and/or one natural extract of vegetable or animal origin, and/or a mixture thereof;
c) a dispersing carrier miscible with water, consisting of a mixture of alcohols and/or polyalcohols and/or glycols and/or polyglycols and/or polyoxyethylenes and a quantity of water of 0 to 25% (v/v), relative to the total volume of said dispersing carrier;
d) additives such as modifying agents and/or surfactants and/or preserving agents and/or a mixture thereof; wherein the components of said vesicles are present in the quantities specified in the following tables (wherein the sum of the single components adds up to 100%):
**a) -Composition of the nanovesicles**
| **Phospholipids** | **Active principles** | **Natural extracts or substances** | **Modifiers and/or surfactants** | **Preservatives** | **Dispersing carrier** |
|---|---|---|---|---|---|
| (% w/v) | (% w/v) | (% w/v) | (% w/v) | (% w/v) | (% w/v) |
| **1 - 45** | **0 - 10** | **0 - 10** | **0 - 10** | **0 - 1** | **60 - 99** |
**b) - Composition of the aqueous dispersing carrier**
| **Alcohols** (% v/v) | **Glycols** (% v/v) | Polyalcohols or polyoxyethylene s (% v/v) | **Water** (% v/v) |
|---|---|---|---|
| **0 - 70** | **0 - 70** | **0 - 70** | **0 - 25** |

2. The three-dimensionally aggregated nanometric vesicles according to claim 1, wherein said at least one phospholipid is selected from the group including phosphatidylcholine, phosphatidylcholine of hydrogenated and non-hydrogenated soy, and derivatives thereof, such as those mentioned in the preceding claim, and/or mixtures thereof.

3. The three-dimensionally aggregated nanometric vesicles according to claim 1 or 2, wherein said at least one active principle is a lipophilic and/or hydrophilic substance selected from the group including antiviral drugs, vitamins, vitamin C, vitamin E, active plant extracts, anti-inflammatory drugs, corticosteroids, antibiotics, fungicides, anti- psoriasis drugs, anesthetics, alkaloids, hydroxy acids, antioxidants and moisturizers, and/or mixtures thereof.

4. The three-dimensionally aggregated nanometric vesicles according to any one of the preceding claims, wherein, in said dispersing carrier miscible with water, the co-solvent mixture is selected from the group including ethanol, isopropyl alcohol, glycerol, ethylene glycol, propylene glycol, all polyethylene glycols (PEG) having different molecular weight, and water; wherein water is present in a quantity equal to 0%, or water is present in a quantity of 1% to 25% (v/v) of the dispersing carrier.

5. A three-dimensional aggregate of nanometric vesicles in accordance with any one of the preceding claims.

6. A method for preparing three-dimensionally aggregated nanometric vesicles in accordance with any one of the preceding claims, said method comprising or consisting of the following steps:
a) preparing, under stirring, a homogeneous mixture of the co-solvents and any additives, including water, if present;
b) mixing, under stirring, the homogeneous mixture obtained in step a) with at least one phospholipid and at least one active principle, until hydration of said phospholipid and formation of a colloidal system comprising said aggregated phospholipid-based nanovesicles are achieved;
c) sonicating said colloidal system obtained in step b) by using 10 to 100 cycles (each cycle consisting of2-5 seconds ON and 2-5 seconds OFF) with an amplitude of 14-15 µm;
wherein all the steps of said method are carried out at temperatures below 30°C.

7. Use of the aggregated nanovesicles according to claims 1 to 5 in order to prepare pharmaceutical and/or cosmetic formulations for topical application.

8. A pharmaceutical composition comprising the three-dimensionally aggregated, phospholipid-based nanometric vesicles according to claims 1 to 5.

9. A composition according to claim 8 for use in the topic treatment of skin disorders.

10. A composition for use according to claim 9, in which said skin disorders are acne and cicatricial alterations, ulcers and or keloid, atopical and seborrheic dermatitis, autoimmune diseases as psoriasis.

11. A composition according to claim 8 or for use according to claims 9-10, in which the active ingredients of the three-dimensionally aggregated, phospholipid-based nanometric vesicles are: clotrimazole, curcumin, and baicalin.

## Patentansprüche

1. Dreidimensional aggregierte nanometrische Vesikel auf Phospholipidbasis, dispergiert in einer alkoholischen Mischung, umfassend oder bestehend aus:
a) mindestens ein Phospholipid, ausgewählt aus einem oder mehreren natürlichen oder synthetischen Phospholipiden, ob rein oder gemischt, ausgewählt aus der Gruppe, umfassend Soja - oder Eilecithin, Phosphatidylcholin in verschiedenen Reinheitsgraden, ob hydriert oder nicht hydriert, Phosphatidylethanolamin, Phosphatidylserin, Phosphatidylglycerin, Phosphatidylinositol, Dimyristoylphosphatidylcholin, Dipalmitoylphosphatidylcholin, Distearoylphosphatidylcholin, Sphyngomyelin, Palmitoylstearoylphosphatidylcholin, hydrierte und nicht hydrierte Phospholipide, die von Soja und/oder Mischungen davon stammen;
b) mindestens einen pharmazeutischen und/oder kosmetischen Wirkstoff, ein biologisch aktives Molekül natürlichen oder synthetischen Ursprungs und/oder einen natürlichen Extrakt pflanzlichen oder tierischen Ursprungs und/oder eine Mischung davon;
c) einen mit Wasser mischbaren dispergierenden Träger, bestehend aus einer Mischung von Alkoholen und/oder Polyalkoholen und/oder Glykolen und/oder Polyglykolen und/oder Polyoxyethylenen und einer Wassermenge von 0 bis 25 % (v/v), bezogen auf die Gesamtvolumen des dispergierenden Trägers;
d) Zusatzstoffe wie Modifizierungsmittel und/oder Tenside und/oder Konservierungsmittel und/oder eine Mischung davon;
wobei die Komponenten der Vesikel in den in den folgenden Tabellen angegebenen Mengen vorhanden sind (wobei die Summe der einzelnen Komponenten zu 100% entspricht):
**a) Zusammensetzung von Nanovesikeln**
| **Phospholipide** (% in Gew./Vol.) | **Wirkstoffe** (% in Gew./vol.) | **Natürliche Extrakte oder Substanzen** (% in Gew,/Vol.) | **Modifikatoren und/oder Tenside** (% in Gew./Vol.) | **Konservierungsmittel** (% in Gew./Vol.) | **Disperersionsträger** (% in Gew./Vol.) |
|---|---|---|---|---|---|
| **1-45** | **0-10** | **0-10** | **0-10** | **0-1** | **60-99** |
**b) Zusammensetzung des wässrigen Dispersionsträgers**
| **Alkohole** (% v/v) | **Glykole** (% v/v) | **Poli-Alkohole bzw. Polyoxyethylene** (% v/v) | **Wasser** (v/v) |
|---|---|---|---|
| **0-70** | **0-70** | **0-70** | **0-25** |

2. Dreidimensional aggregierte nanometrische Vesikel nach Anspruch 1, wobei das mindestens eine Phospholipid aus der Gruppe ausgewählt ist, die Phosphatidylcholin, Phosphatidylcholin von hydriertem und nicht hydriertem Soja und Derivate davon, wie die im vorhergehenden Anspruch erwähnten, und/oder Mischungen davon.

3. Dreidimensional aggregierte nanometrische Vesikel nach Anspruch 1 oder 2, wobei der mindestens eine Wirkstoff eine lipophile und/oder hydrophile Substanz ist, ausgewählt aus der Gruppe umfassend antivirale Arzneimittel, Vitamine, Vitamin C, Vitamin E, aktive Pflanzenextrakte, Anti - Entzündungsmedikamente, Corticosteroide, Antibiotika, Fungizide, Mittel gegen Psoriasis, Anästhetika, Alkaloide, Hydroxysäuren, Antioxidantien und Feuchtigkeitscremes und/oder Mischungen davon.

4. Dreidimensional aggregierte nanometrische Vesikel nach einem der vorangehenden Ansprüche, wobei in dem mit Wasser mischbaren dispergierenden Träger das Co-Lösungsmittel-Gemisch aus der Gruppe ausgewählt ist, die Ethanol, Isopropylalkohol, Glycerin, Ethylenglycol, Propylenglycol, alle Polyethylen-Glykole (PEG) mit unterschiedlichem Molekulargewicht und Wasser; wobei Wasser in einer Menge gleich 0 % vorhanden ist oder Wasser in einer Menge von 1 % bis 25 % (v/v) des dispergierenden Trägers vorhanden ist.

5. Dreidimensional aggregierte nanometrische Vesikel nach einem der vorangehenden Ansprüche.

6. Verfahren zur Herstellung dreidimensional aggregierter nanometrischer Vesikel nach einem der vorhergehenden Ansprüche, wobei das Verfahren die folgenden Schritte umfasst oder daraus besteht:
a) Herstellen, durch Rühren, einer homogenen Mischung aus den Co-Lösungsmitteln und jeglichen Additiven, einschließlich Wasser, falls vorhanden;
b) Mischen, unter Rühren, der in Schritt a) erhaltenen homogenen Mischung mit mindestens einem Phospholipid und mindestens einem Wirkstoff, bis die Hydratation des Phospholipids und die Bildung eines kolloidalen Systems, das die aggregierten Nanovesikel auf Phospholipidbasis umfasst, erreicht sind;
c) Beschallen des in Schritt b) erhaltenen kolloidalen Systems unter Verwendung von 10 bis 100 Zyklen (jeder Zyklus besteht aus 2-5 Sekunden EIN und 2-5 Sekunden AUS) mit einer Amplitude von 14-15 µm;
wobei alle Schritte des Verfahrens bei Temperaturen unter 30°C, im Wesentlichen bei Raumtemperatur, durchgeführt werden.

7. Verwendung der aggregierten Nanovesikel nach den Ansprüchen 1 bis 5 zur Herstellung pharmazeutischer und/oder kosmetischer Formulierungen zur topischen Anwendung.

8. Pharmazeutische und/oder kosmetische Zusammensetzung, umfassend die dreidimensional aggregierte Nanovesikel nach den Ansprüchen 1 bis 5.

9. Zusammensetzung nach Anspruch 8 zur Verwendung bei der Behandlung von Hauterkrankungen.

10. Zusammensetzung nach Anspruch 9, wobei die Hauterkrankungen Akne und Narbenveränderungen, Geschwüre und/oder Keloide, atopische und seborrhoische Dermatitis, Autoimmunerkrankungen wie Psoriasis.

11. Zusammensetzung nach Anspruch 8 zur Verwendung nach Ansprüchen 9-10, wobei die Wirkstoffe der dreidimensional aggregierten Phospholipid enthaltenen Nanovesikel sind: Clotrimazol, Curcumin und Baicalin.

## Revendications

1. Vésicules nanométriques agrégées en trois dimensions à base de phospholipides dispersées dans un mélange alcoolique comprenant ou consistant en:
a) au moins un phospholipide choisi parmi un ou plusieurs phospholipides naturels ou synthétiques, purs ou en mélange, choisis dans le groupe comprenant la lécithine de soja ou d'œuf, la phosphatidylcholine à différents degrés de pureté, hydrogénée ou non, la phosphatidyléthanolamine, la phosphatidylsérine, le phosphatidylglycérol, le phosphatidylinositol, la dimyristoylphosphatidylcholine, la dipalmitoylphosphatidylcholine, la distéaroylphosphatidylcholine, la sphyngomyéline, la palmitoylstéaroylphosphatidylcholine, les phospholipides hydrogénés et non hydrogénés issus du soja et/ou leurs mélanges;
b) au moins un principe actif pharmaceutique et/ou cosmétique, une molécule biologiquement active d'origine naturelle ou synthétique, et/ou un extrait naturel d'origine végétale ou animale, et/ou leur mélange;
c) un support dispersant miscible à l'eau, constitué d'un mélange d'alcools et/ou de polyalcools et/ou de glycols et/ou de polyglycols et/ou de polyoxyéthylènes et d'une quantité d'eau de 0 à 25 % (v/v), par rapport au volume total dudit support dispersant;
d) des additives comme des agents modifiés et/ou des surfactants et/ou des agents conservateurs et/ou un mélange de ceux-ci;
dans lesquels les composants desdites vésicules sont présents dans les quantités spécifiées dans les tableaux suivants (dans lesquelles la somme des composants individuels correspond à 100):
**a) Composition des nanovésicules**
| Phospholipides (% in poids/v) | Principles actifs (% en poids/v) | Extraits ou substances naturelles (% en poids/v) | Modifieurs et/ou tensioactifs (% en poids/v) | Conservants (% en poids/v) | Support de dispersion (% en pois/v) |
|---|---|---|---|---|---|
| 1-45 | 0-10 | 0-10 | 0-10 | 0-1 | 60-99 |
**b) Composition du support de dispersion aqueux**
| Alcools (% v/v) | Glycols (% v/v) | Polyalcools ou Polyoxyéthylènes S (% v/v) | Eau (% v/v) |
|---|---|---|---|
| 0-70 | 0-70 | 0-70 | 0-25 |

2. Vésicules nanométriques agrégées en trois dimensions selon la revendication 1, dans lesquelles ledit au moins un phospholipide est choisi dans le groupe comprenant la phosphatidylcholine, la phosphatidylcholine de soja hydrogénée et non hydrogénée, et leurs dérivés, tels que ceux mentionnés dans la revendication précédente, et/ ou leurs mélanges.

3. Vésicules nanométriques agrégées en trois dimensions selon la revendication 1 ou 2, dans lesquelles ledit au moins un principe actif est une substance lipophile et/ou hydrophile choisie dans le groupe comprenant les médicaments antiviraux, les vitamines, la vitamine C, la vitamine E, les extraits actifs de plantes, les médicaments anti-inflammatoires, les corticostéroïdes, les antibiotiques, les fongicides, les médicaments anti-psoriasis, les anesthésiques, les alcaloïdes, les hydroxyacides, les antioxydants et hydratants, et/ou leurs mélanges.

4. Vésicules nanométriques agrégées en trois dimension selon l'une des revendications précédentes, dans lesquelles, dans ledit support dispersant miscible à l'eau, le mélange des cosolvants est choisi dans le groupe comprenant l'éthanol, l'alcool isopropylique, le glycérol, l'éthylène glycol, le propylène glycol, tous les polyéthylène glycols (PEG) de masse moléculaire différente, et de l'eau; dans laquelle l'eau est présente en une quantité égale à 0%, ou l'eau est présente en une quantité de 1 % à 25 % (v/v) du support dispersant.

5. Agrégat en trois dimensions de vésicules nanométriques selon l'une des revendications précédentes.

6. Procédé de préparation de vésicules nanométriques agrégées en trois dimensions selon l'une des revendications précédentes, ledit procédé comprenant ou consistant en les étapes suivantes :
a) préparer, sous agitation, un mélange homogène des co-solvants et de tout additif, y compris de l'eau, si elle est présente;
b) mélanger, sous agitation, le mélange homogène obtenu à l'étape a) avec au moins un phospholipide et au moins un principe actif, jusqu'à hydratation dudit phospholipide et formation d'un système colloïdal comprenant lesdites nanovésicules agrégées à base de phospholipides;
c) sonication dudit système colloïdal obtenu à l'étape
b) en utilisant 10 à 100 cycles (chaque cycle consistant en 2-5 secondes ON et 2-5 secondes OFF) avec une amplitude de 14-15 µm;
dans lequel toutes les étapes dudit procédé sont réalisées à des températures inférieures à 30°C.

7. Utilisation des nanovésicules agrégées selon les revendications 1 à 5 pour préparer des formulations pharmaceutiques et/ou cosmétiques à application topique.

8. Composition pharmaceutique comprenant les nanovésicules agrégées en trois dimensions à base de phospholipides selon les revendications 1 à 5.

9. Composition selon la revendication 8 pour une utilisation dans le traitement des désordres cutanés.

10. Composition pour utilisation selon la revendication 9, dans laquelle les désordres cutanés sont de l'acné et des changements cicatriciels, des ulcères et de la dermatite chéloïde, atopique et séborrhéique, et de maladies auto-immunes telles que le psoriasis.

11. Composition selon la revendication 8 pour une utilisation selon les revendications 9-10, dans laquelle les ingrédients actifs des nanovésicules agrégées en trois dimensions à base de phospholipides sont : clotrimazole, curcumine et baicaline.
